Europäisches Patentamt

European Patent Office

Office européen des brevets

(11) Publication number: **0 416 863 A1**

# EUROPEAN PATENT APPLICATION

(21) Application number: **90309652.7**

(22) Date of filing: **04.09.90**

(30) Priority: **04.09.89 GB 8919912**

(43) Date of publication of application:
**13.03.91 Bulletin 91/11**

(84) Designated Contracting States:
**AT BE CH DE DK ES FR GB GR IT LI LU NL SE**

(51) Int. Cl.5: **A61B 19/00, A61B 8/08**

(71) Applicant: **Wickham, John Ewart Alfred**
**29 Devonshire Place**
**London W1N 1PE(GB)** ,

(72) Inventor: **Wickham, John Ewart Alfred**
**29 Devonshire Place**
**London, W1N 1PE(GB)**
Inventor: **Davies, Brian Lawrence**
**37 Burnaby Gardens**
**Chiswick, London W4 3DR(GB)**
Inventor: **Hibberd, Roger David**
**53 Woodsome Road**
**London, NW5 1SA(GB)**
Inventor: **Timoney, Anthony Gererd**
**The Institute of Urology, 172 Shaftesbury Avenue**
**London, WC2H 8JE(GB)**

(74) Representative: **Thiemann, Peter Albert William et al**
**LLOYD WISE, TREGEAR & CO. Norman House 105-109 Strand**
**London WC2R 0AE(GB)**

(54) **Frame for medical implement.**

(57) A frame is described for positioning and guiding an implement with which a surgical or diagnostic procedure may be carried out, particularly a resectoscope. The frame comprises an annular frame member (10,50) and an annular ring (15,57) held captive and rotatable about its axis in a plane parallel to that of the frame member. An arcuate bow (18,65) extends across the ring and carries a mounting block (20,66) for the implement, the movement of which is controlled by moving the block along the bow and by moving the ring relative to the frame member. The frame has means for mounting it in a desired position relative to a patient. If desired the movements of the block and the ring can be motorise under the control of a computer programme.

Fig.1

This invention relates to a frame for positioning and guiding an implement with which a surgical or diagnostic procedure may be carried out. The frame of the present invention is particularly useful for holding and guiding a resectoscope or tissue disintegrating device, such as that described in the specification of EP-A-88303138.7 for use, for example, when carrying out non-invasive surgical treatment of the prostate. However, the frame may also be used for positioning and guiding lasers for heating tissue or ultrasound devices.

In the non-invasive surgical treatment of the prostate a resectoscope or like device is inserted through the urethra and prostate material is removed to leave an orifice in the prostate. It is, however, time consuming to monitor the progress of the operation as the resection has periodically to be halted so that a check on position can be made.

In other non-invasive surgical procedures, it may be necessary to hold a medical implement in a given position relative to a patient for a period of time or to hold the implement in a number of different positions in a given period of time.

It is an object of the present invention to provide a frame for holding and guiding a medical implement in such a way that the implement can be moved through controlled incremental motions or held in a predetermined position relative to a patient so as to facilitate a number of surgical procedures.

According to the present invention there is provided a frame for positioning and guiding a medical implement characterised by an annular frame member, an annular ring mounted adjacent the frame member, means for holding the ring captive relative to the frame member such that the ring is rotatable about its axis in a plane parallel to the plane of the frame member, an arcuate bow extending across the ring, a mounting block for the medical implement mounted on said bow and capable of being moved along the bow, and means whereby said frame member can be held in a desired position relative to a patient.

The radius of the ring, the radius of curvature of the bow and the distance of the tip of the medical implement from the bow are selected such that full movement of the implement around the ring and across the bow will sweep out two substantially conical regions on the side of the ring remote from the bow, one of which regions tapers from the ring to an apex and the other and smaller of which extends away from the apex. In the case where the medical implement is a resectoscope, the smaller conical region is that of the material to be removed to leave a conical orifice in the prostate. The base of this latter cone will be located at the neck of the bladder and the apex will be located at the veru montanum.

The present frame thus holds and guides the resectoscope to provide the appropriate motion to sweep out the required conical orifice in the prostate and at the same time limits the maximum travel of the resectoscope, thus avoiding damage to adjacent tissue or the removal of too much prostate material. With the frame it is possible to provide controlled increments of cut and a gradual increase in the size of the required conical shape up to the desired maximum. Furthermore, it is possible to know where the cutter of the resectoscope is located inside the patient merely by examination of the position of the ring and mounting means relative to the frame. Finally, the frame provides safety for the patient since even if the surgeon's hand were to slip, nothing outside the permitted conical shape would be cut.

If desired, variable stops may be provided on the frame to allow for smaller quantities of tissue to be removed.

In order to enable the invention to be more readily understood, reference will now be made to the accompanying drawings, which illustrate diagrammatically and by way of example an embodiment thereof, and in which:-

Figure 1 is a side view of a frame for holding and guiding a resectoscope, the frame being in an operating position,

Figure 2 is a front view of the frame in an upright position with some parts removed,

Figure 3 is a side view of a resectoscope,

Figure 4 is a diagram showing in section the region swept by the resectoscope,

Figure 5 is an end view of the swept region at the neck of the bladder,

Figure 6 is a front view of a modified frame for holding and guiding a resectoscope,

Figure 7 is a side view of the frame shown in Figure 6,

Figure 8 is an end view of the frame shown in Figures 6 and 7, and

Figure 9 is another end view of the frame.

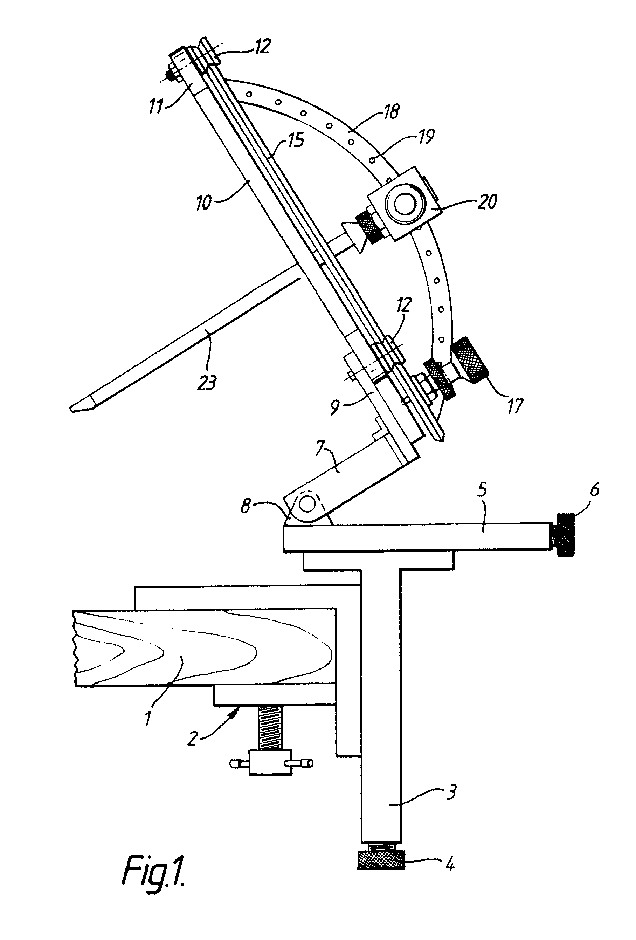

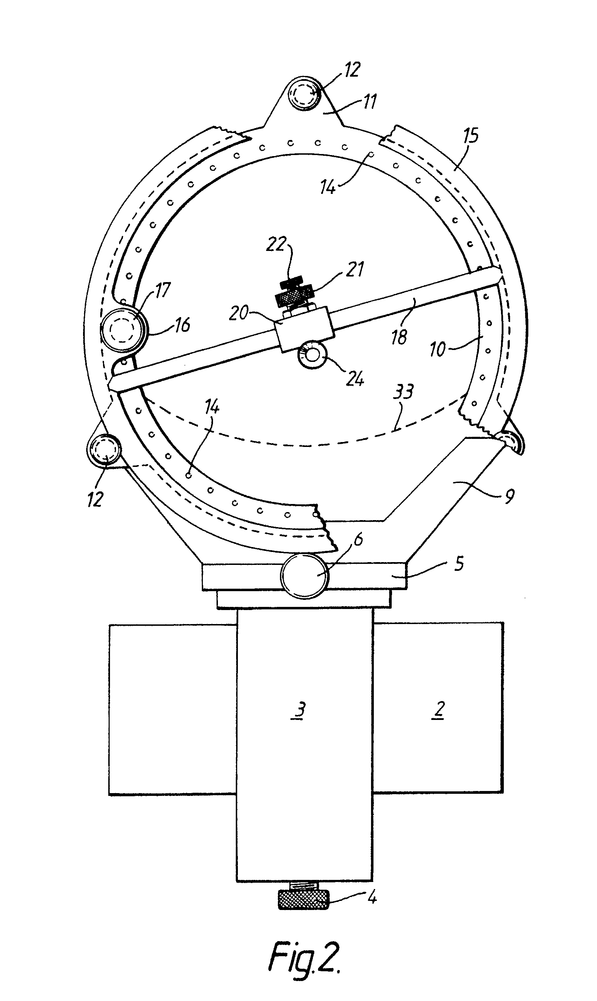

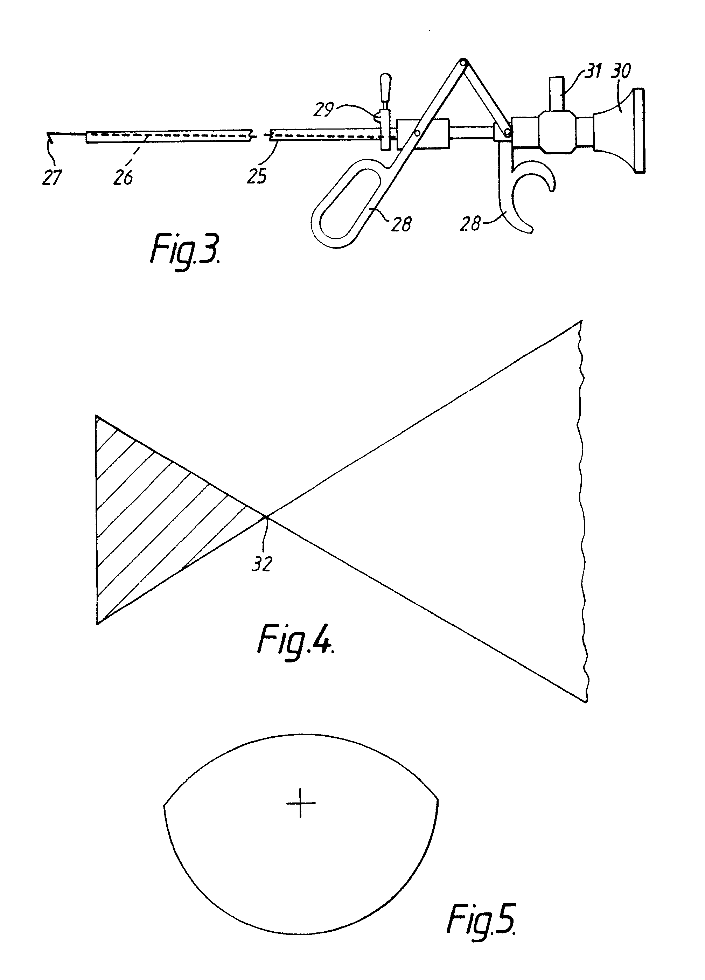

Referring now to Figures 1 and 2 of the drawings, there is shown a frame for holding and guiding a resectoscope such as that shown in Figure 3.

The frame is mounted on a table 1 by means of a clamp 2 to which is mounted a vertical adjustment table 3 which can be moved up and down relative to the clamp by a screw operable by a knurled knob 4. A horizontal adjustment table 5 is mounted on top of the table 3 and the table 4 can be moved in a horizontal direction by a screw operable by a knurled knob 6. Two arms 7 are mounted on blocks 8 at one end of the table 5 so as to be pivotable and a locking screw (not shown) is provided for locking the arms in a pivoted position. At their free ends, the arms 7 are fixedly connected to a bifurcated support plate 9. An annular frame 10 is fixed to the support plate and at three equiangularly spaced regions is provided with extensions 11 each of which carries a roller 12 formed with a V-groove, the rollers being mounted for rotation in low friction bearings. The frame is formed with a plurality of equally spaced holes or depressions 14 for a purpose to be explained hereinafter.

An annular ring 15 with a tapered outer rim is held captive by the rollers 12 and is freely rotatable in a plane parallel to the plane of the frame 10. The ring 15 has an inwardly directed extension 16 on which is mounted a locking device with a knurled knob 17. The knob 17 controls a spring-loaded ball which can engage in the holes or depressions 14 to lock the ring 15 relative to the frame 10. The ball can be disengaged from a hole 14 by lifting the knob to permit rotation of the ring 15.

An arcuate bow 18 extends diametrically across the ring 15 and carries a mounting for a resectoscope, the bow 18 also being formed with a plurality of equally spaced holes or depressions 19. The mounting comprises a block 20 housing a spring-loaded ball which can engage in the holes or depressions 19 to lock the block and a knurled knob 21 for disengaging the ball from a hole or depression 19 to permit movement of the block along the bow. The knob 21 can either be screwed fully home to lock the block 20 relative to the bow 18 or only partially screwed home to allow a variable amount of friction between the bow and the block. An additional locking screw is also provided for locking the knob 21 and is operable by another knurled knob 22.

The mounting block 20 also carries an elongated tubular sheath or endoscope 23 for holding the resectoscope, the sheath having a funnel shaped opening 24 to guide the resectoscope.

A suitable resectoscope is shown in Figure 3 and comprises a hollow tube 25 mounting electrical wires 26 for a tungsten wire resector 27 mounted at the end of the tube. The resectoscope also carries a scissors mechanism 28 for moving the tube 25 to cover or uncover the resector 27, a locking ring 29 for locking the resectoscope on the mounting block 20, an eyepiece 30 incorporating a telescope and an inlet 31 for irrigation liquid.

When the annular ring 15 is rotated and the mounting block 20 is traversed across the bow 18, the end of a resectoscope, when in position, will sweep out a substantially conical region as shown hatched in Figure 4. If a shaped guard 33 of metal or plastics is clipped on to the ring 10 then the end view of the base of the swept region will be as shown in Figure 5.

In the operation of the frame of the present invention, a patient for a prostatectomy is placed on an operating table adjacent the frame. The surgeon then adjusts the position of the frame by adjustment of the vertical table 3, the horizontal table 5 and the angle of the arms 7 until he adjudges that, when the resectoscope is swept round the conical region, the apex 32 of the region lies at the veru montanum of the patient. He then checks the position using the endoscope and makes whatever fine adjustments to the tables 3 and 5 and the arms 7 may be necessary to ensure that the apex 32 lies at the veru montanum.

The surgeon then commences to operate the resectoscope and by moving the ring 15 around its axis from one hole or depression 14 to the next, by traversing the mounting block 20 back and forth across the bow and by increasing the depth of insertion of the resectoscope the required conical region of prostate material between the veru montanum and the neck of the bladder will be removed. The operation can be conducted more quickly than heretofore as the surgeon does not have to monitor the removal of prostate material from time to time. During the operation, the site of the operation is irrigated by the introduction of irrigation liquid through the inlet 31. Normally this liquid will collect in the patient's bladder and under certain circumstances it may become necessary to drain the bladder during the course of the operation, although this is not usually required.

It will be appreciated that many modifications of the present frame may be made. In particular, it may be more convenient to secure the frame to an overhead mounting rather than to the tables 3 and 5 and to provide In addition it is possible to provide motorised drives for the rotation of the annular ring 15, the traverse of the block 20 across the bow 18, and the cutting stroke of the resector 27. Furthermore, it is be possible to motorise the linear actuation of the whole of the resectoscope to provide a variable increase in the length of the conical region which is resected.

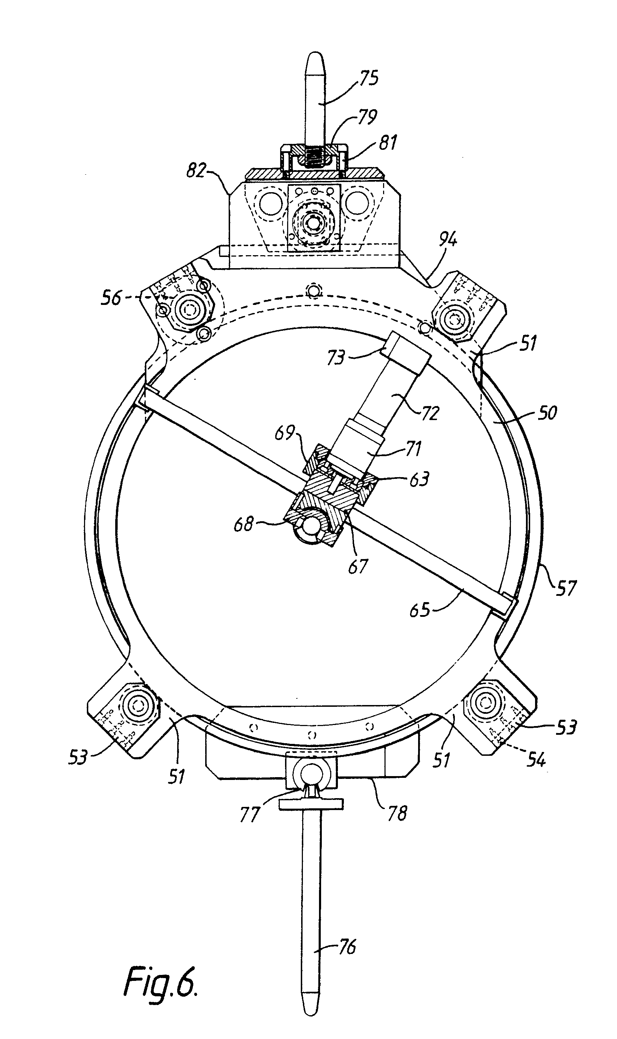

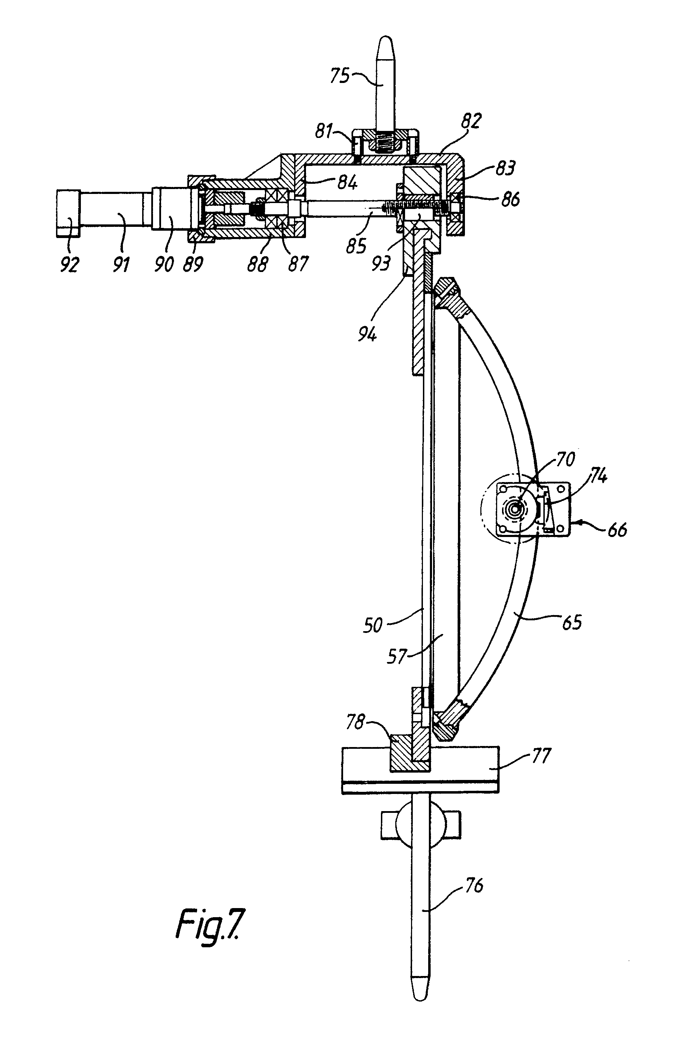

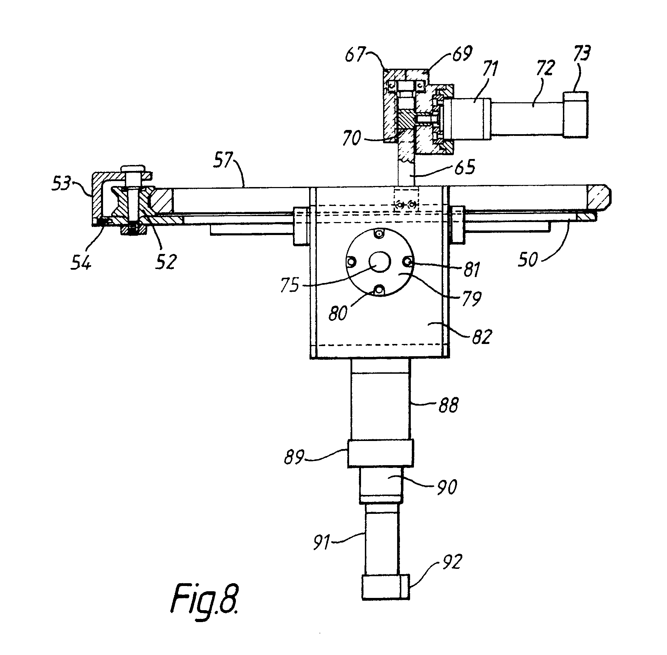

Referring now to Figures 6 to 9 of the drawings, there is shown a modification of the frame in which motor drives are provided for rotating the annular ring, traversing an implement mounted across the bow and for operating the implement.

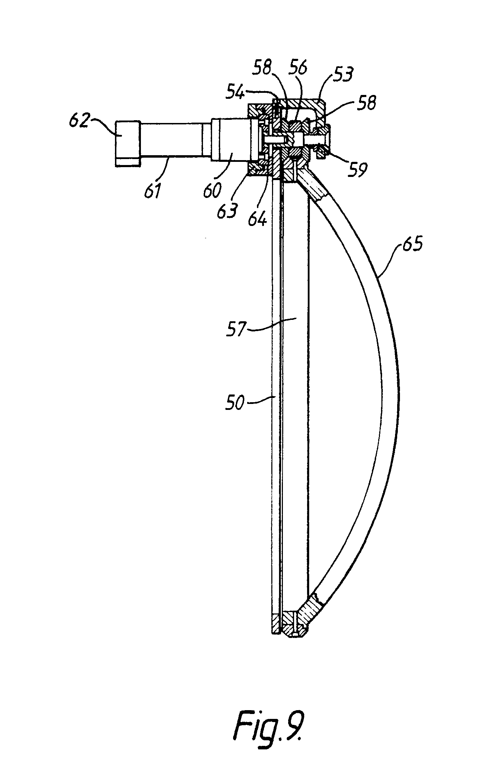

As with the embodiment of Figures 1 and 2, the frame of Figures 6 to 9 comprises an annular frame 50 having three extensions 51 each of which carries a roller 52 formed with a groove, the rollers being mounted for rotation in low friction bearings and held by support brackets 53 secured by screws 54. A fourth extension 55 of the annular frame carries a pinion 56 which meshes with gear teeth (not shown) formed on the outside edge of an annular ring 57 which has a tapered outer rim and which is held by the rollers in a plane parallel to the plane of the frame 50. The three rollers 52 support the ring 57 but the pinion is capable of rotating the ring about its axis by engagement of the pinion with the gear teeth. As shown in Figure 9, the pinion 56 is held between support rollers 58.

A shaft 59 for the pinion is connected through a gearbox 60 to a motor 61 controlled by an encoder 62, the gearbox, motor and encoder being held in place by a locknut 63 engaging in a face plate 64. This arrangement permits the ring 57 to be rotated relative to the annular frame by applying a signal through the encoder 62 to the motor 61.

The frame of Figures 6 to 9 also has an arcuate bow 65 which extends substantially diametrically across the ring 57. A mounting block 66 carried on the ring includes a carriage 67 and brackets 68 for holding a resectoscope or other implement which will not be described here in future detail, and includes a carriage 69 for supporting a motor drive. The inner surface of the bow 65 is formed with gear teeth (not shown) engaged by a pinion 70 which is drivable through a gear box 71 by a motor 72 controlled by an encoder 73. This arrangement permits the resectoscope or other implement to be traversed across the bow 65. In order to prevent the implement from wobbling during its traverse across the bow and in order to take up the slack, the carriage 69 is provided with wedge shaped pressure pads 74 of nylon or other plastics. In a modification of the frame, the outer surface of the bow 65 may be formed with gear teeth as this may slightly increase the range of travel of the implement.

While the frame of Figures 1 and 2 was mounted adjacent and clamped to a table, the frame of Figures 6 to 9 is intended to be supported above a table by being suspended indirectly from a frame which straddles a patient. To this end, one end of the frame carries a support spigot 75 which is intended to be received in a ball-shaped universal joint which is supported from the frame which straddles a patient, either directly or through one or more arms and similar joints. At its other end, the frame has another support spigot 76 which may be held in a mounting on an operating table. The spigot 76 is mounted, with play, in a linear guide 77 which is capable of sliding relative to a base block 78.

The support spigot 75 is bolted to a disc 79 formed with notches 80 in which engage screws 81 connecting the disc to a bracket 82 having a top portion 83 engaging over the frame and a bottom portion 84 engaging under the frame. A shaft 85 has one end received in a bearing 86 in the top portion 83 while the other end is supported by bearings 87 in a bearing housing 88 attached to the bottom portion 84 of the bracket 82. The shaft 85 is drivable by a drive unit coupled to the bearing housing by a locknut 89, the drive unit comprising a gearbox 90, motor 91 and encoder 92.

The shaft 85 is threaded and engages in a threaded nut 93 captive at the end of a stiffening member 94 fixed to the annular frame 50. Operation of the motor 91 causes rotation of the shaft 85 and thereby movement of the frame relative to the spigot 75 for fine adjustment of the position of the frame.

The operation of the frame shown in Figures 6 to 9 is similar to that of the frame shown in Figures 1 and 2 and the frame may be used with a resectoscope similar to that shown in Figure 3 or with any other appropriate medical implement. However, in the operation of the frame shown in Figures 6 to 9 the encoders 62, 73 and 92 are all connected to a computer (not shown) via a servo-board (also not shown) to provide a standard control loop for controlling the positioning of the resectoscope or other implement relative to the frame. In the operation of this standard control loop, the encoders 62, 73 and 92 serve to measure the position of their respective motor shafts, the encoders generating pulses which are counted by the servo-board, the rate of pulses giving the velocity of rotation of the motors. From this information the computer works out where the respective motor is relative to a desired position and sends a signal to the servo-bboard which applies a signal to the motor. Such a control loop system is well known and will not be described in detail.

The computer is supplied with a programme such that, if the implement is a resectoscope, the desired cuts are made over the swept region. If however, the implement is a laser or ultrasound instrument, the programmes may be chosen so that the implement is held in a desired position for a predetermined period of time and then moved to another position for another predetermined period of time.

While the frame has been particularly described for use in prostatectomy, it is to be appreciated that it will find use for other operations where a resectoscope or other instrument has to move through a predetermined region. In such a case, the shape of the annular frame 10 or 50 and annular ring 15 or 57 may be adapted to required use or stops may be mounted on the frame or ring and/or on the bow 18 or 65 to restrict the swept region.

Thus the frame may be used where the sheath 23 and resectoscope or other implement are inserted through a natural body orifice as in non-invasive surgery or where the sheath and resectoscope or other implement are inserted through a percutaneous access site. In each case, the frame permits the operation to be performed more quickly and with greater safety for the patient.

## Claims

1. A frame for positioning and guiding a medical implement characterised by an annular frame member, an annular ring mounted adjacent the frame member, means for holding the ring captive relative to the frame member such that the ring is rotatable about its axis in a plane parallel to the plane of the frame member, an arcuate bow extending across the ring, a mounting block for the medical implement mounted on said bow and capable of being moved along to the bow, and means whereby said frame member can be held in a desired position relative to a patient.

2. A frame as claimed in Claim 1, wherein locking means are provided for locking the ring relative to the annular frame member and locking means are provided for locking the mounting block relative to the bow, both such locking means being releasable to permit movement of the annular ring relative to the frame member and movement of the mounting block and thus the medical implement across the bow.

3. A frame as claimed in Claim 1 or 2, wherein the radius of the ring, the radius of curvature of the bow and the distance of the tip of the medical implement from the bow are selected such that full movement of the implement around the ring and across the bow will sweep out two substantially conical regions on the side of the ring remote from the bow, one of which regions tapers from the ring to an apex and the other and smaller of which extends away from the apex.

4. A frame as claimed in Claim 3, wherein stops are provided to restrict the swept region.

5. A frame as claimed in any one of Claims 1 to 4, wherein motorised drives are provided for rotating the ring relative to the annular frame and for translating the mounting block along the arcuate bow, and wherein each drive includes a motor arranged to drive a pinion, there being gear teeth formed in the rim of the annular ring and on the arcuate bow for engagement by a respective pinion.

6. A frame as claimed in Claim 5, wherein each motor is controlled by an encoder, and wherein the encoders can be connected with a computer in a standard control loop system to permit computer-controlled movement of the medical implement.

*Fig.1.*

*Fig.2.*

Fig.3.

Fig.4.

Fig.5.

Fig.6.

Fig.7.

Fig.8.

*Fig.9.*

European
Patent Office

**EUROPEAN SEARCH
REPORT**

Application Number

**EP 90 30 9652**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | FR-A-1 056 372 (CHEVALIER)<br>* Page 2, column 1, lines 2-23; figure 2 *<br>– – – | 1 | A 61 B 19/00<br>A 61 B 8/08 |
| Y | US-A-4 608 977 (BROWN)<br>* Column 4, lines 21-68; column 7, lines 35-41; column 9, lines 48-59; abstract; figures 1,3 *<br>– – – | 1 | |
| A | | 2,5,6 | |
| A | US-A-4 335 715 (KIRKLEY)<br>* Column 3, lines 47-63; column 4, lines 10-24; figure 1 *<br>– – – | 2 | |
| A | US-A-4 350 159 (GOUDA)<br>* Abstract; column 2, lines 19-22; figures 1-4 *<br>– – – | 5 | |
| A | US-A-4 638 798 (SHELDEN)<br>– – – | | |
| A | US-A-2 393 982 (GIESEN)<br>– – – | | |
| A,D | EP-A-0 286 415 (WICKHAM)<br>– – – – – | | |

TECHNICAL FIELDS
SEARCHED (Int. Cl.5)

A 61 B

The present search report has been drawn up for all claims

| Place of search | Date of completion of search | Examiner |
|---|---|---|
| The Hague | 26 November 90 | KLEIN C. |